(19) Europäisches Patentamt
European Patent Office
Office européen des brevets

(11) Veröffentlichungsnummer: **0 050 777**
**A2**

(12) # EUROPÄISCHE PATENTANMELDUNG

(21) Anmeldenummer: **81108191.8**

(22) Anmeldetag: **12.10.81**

(51) Int. Cl.³: **C 07 C 69/74**
C 07 C 67/475, C 07 C 69/63
C 07 C 49/457
//C07C121/75, A01N53/00

(30) Priorität: **23.10.80 DE 3040001**
**21.11.80 DE 3043975**

(43) Veröffentlichungstag der Anmeldung:
**05.05.82 Patentblatt 82/18**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Lantzsch, Reinhard, Dr.**
**Heymannstrasse 32**
**D-5090 Leverkusen(DE)**

(72) Erfinder: **Hagemann, Hermann, Dr.**
**Kandinskystrasse 52**
**D-5090 Leverkusen(DE)**

(54) **Verfahren zur Herstellung von chlorfluoralkylsubstituierten Cyclopropancarbonsäuren und deren Derivaten und Zwischenprodukte dafür.**

(57) Die Erfindung betrifft ein Verfahren zur Herstellung von Chlorfluoralkylsubstituierten Cyclopropancarbonsäuren der Formel I

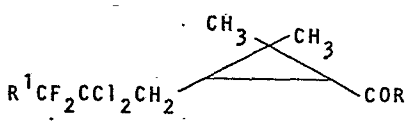

(I)

in welcher
$R^1$ für Fluor oder $CF_3$ steht und
R für OH, Halogen, $C_{1-4}$-Alkoxy steht,
deren Derivaten, indem man chlor-fluor-alkylsubstituierte Halogencyclobutanone der Formel II

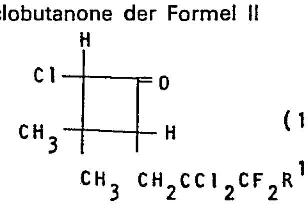

(II)

in welcher $R^1$ die in der Beschreibung angegebene Bedeutung hat, in wasserhaltigen aprotischen organischen Verdünnungsmitteln bei −20 bis +40°C mit wäßrigen Basen umsetzt und aus den dabei erhaltenen Salzen die freie Säure bzw. deren Derivate in üblicher Weise herstellt.

EP 0 050 777 A2

Croydon Printing Company Ltd.

0050777



BAYER AKTIENGESELLSCHAFT 5090 Leverkusen, Bayerwerk

Zentralbereich
Patente, Marken und Lizenzen Rt-by-c
IVa/ZP

Verfahren zur Herstellung von chlorfluoralkylsubstituierten Cyclopropancarbonsäuren und deren Derivaten und Zwischenprodukte dafür

Die Erfindung betrifft ein Verfahren zur Herstellung von chlorfluoralkylsubstituierten Cyclopropancarbonsäuren und deren Derivaten und neue Zwischenprodukte zur Durchführung dieses Verfahrens.

Es ist bereits bekannt geworden, daß man 3-(2'2'-Dichlorvinyl)-2,2-dimethylcyclopropancarbonsäure bzw. deren Ester erhält, indem man 2-(2',2',2'-trichlorethyl)-4-halogencyclobutan-1-one in Gegenwart von Basen in Wasser bzw. Alkohol erhitzt. Es kommt dabei zur Ringverengung und Dehydrohalogenierung in der Seitenkette (DE-OS 2 813 337).

Es war wünschenswert diese Reaktion auch zur Herstellung von chlorfluoralkylsubstituierten Cyclopropancarbonsäuren zu benutzen. Dabei zeigt sich,

daß sich die Cyclobutanone unter dem Einfluß der Chlorfluoralkyl-Seitenkette anders verhalten als unter dem Einfluß der Chloralkyl-Seitenkette. So kann 2-(2',2',2'-Trichlorethyl)-4-chlorcyclobutan-1-on durch Umsetzung mit wäßrigen Basen oder Alkoholen und anschließendes Erhitzen auf über 40°C in Permethrinsäure oder deren Ester übergeführt werden. Diese Reaktion führt bei 2-Chlor-fluoralkyl-substituierten-4-chlorcyclobutan-1-onen zu Produkten, die kein Fluor mehr in der Seitenkette tragen bzw. die sowohl in der Seitenkette als auch im Cyclobutanonring (4-Stellung) substituiert sind.

Das heißt eine Ringverengung führt zu unerwünschten Produkten (andere Seitenkette) oder tritt gar nicht ein.

Außerdem kommt es bei der Durchführung der Reaktion in Alkoholen im Gegensatz zu den trichlorethylsubstituierten Halogencyclobutanonen nicht zur Bildung der entsprechenden Cyclopropancarbonsäureester, sondern zur Bildung alkoxysubstituierter Cyclobutanone. Auch dies zeigt, daß die Chlor-fluor-alkyl-Seitenkette einen wesentlichen Einfluß auf den Gesamtverlauf der Reaktion hat.

1. Es wurde gefunden, daß man chlor-fluor-alkylsubstituierte Cyclopropancarbonsäuren und deren Derivate der Formel I

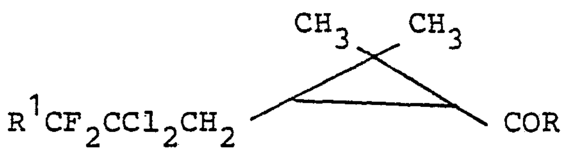

(I)

in welcher

$R^1$ für Fluor oder $CF_3$ steht und

R für OH, Halogen, $C_{1-4}$-Alkoxy steht,

erhält, wenn man chlor-fluor-alkylsubstituierte Halogencyclobutanone der Formel II

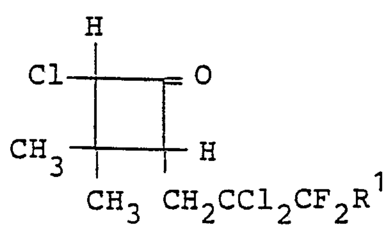

(II)

in welcher

$R^1$ die oben angegebene Bedeutung hat,

in wasserhaltigen aprotischen organischen Verdünnungsmitteln bei -20 bis +40°C mit wäßrigen Basen umsetzt und aus den dabei erhaltenen Salzen die freie Säure bzw. deren Derivate in üblicher Weise herstellt.

1a. Chlor-fluor-alkylsubstituierte Cyclopropancarbonsäuren und ihre Derivate der Formel I in welcher $R^1$ für $CF_3$ steht und $R^2$ die oben angegebene Bedeutung hat sind neu. Sie sind besonders bevorzugt, da sie wichtige Zwischenstufen bei der Herstellung guter Schädlingsbekämpfungsmittel sind.

2. Es wurden die neuen chlor-fluoralkyl-substituierten Halogencyclobutanone der Formel II gefunden

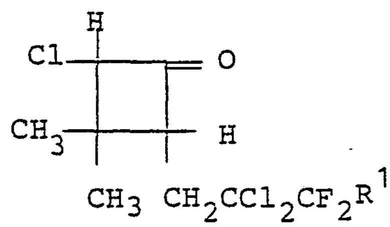

(II)

in welcher

$R^1$ für Fluor oder $CF_3$ steht.

3. Es wurde ferner gefunden, daß man die neuen Cyclobutanone der Formel II enthält, indem man die Cyclobutanone der Formel III

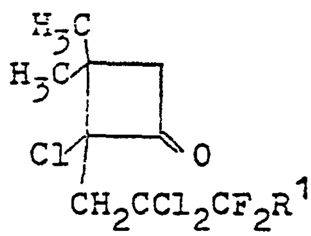

(III)

in welcher

$R^1$ die oben angegebene Bedeutung besitzt,

in Gegenwart eines quartären Ammoniumsalzes erhitzt.

4. Es wurden ferner die neuen Cyclobutanone der Formel III gefunden.

5. Es wurde ferner gefunden, daß man die neuen Cyclobutanone der Formel III erhält, indem man Isobutylen mit einem Säurehalogenid der Formel IV

$$R^1-CF_2-CCl_2-CH_2-CHCl-CO-X \qquad (IV)$$

in welcher

$R^1$ für Fluor oder Trifluormethyl steht und

X Chlor oder Brom bedeutet,

in Gegenwart eines Verdünnungsmittels und in Gegenwart eines tertiären Amins zur Umsetzung bringt.

6. Es wurden ferner die neuen Säurehalogenide der Formel IV gefunden.

7. Es wurde ferner gefunden, daß man die neuen Säurehalogenide der Formel IV erhält, indem man Verbindungen der Formel V

$$R^1-CF_2-CCl_3 \qquad (V)$$

in welcher

$R^1$ die oben angegebene Bedeutung hat,

in Gegenwart von Katalysatoren an Acrylsäure addiert und die entstandene Säure in üblicher Weise in das Halogenid (X = Cl, Br) überführt.

Die chlorfluoralkylsubstituierten Cyclopropancarbonsäuren der Formel I, in welcher

$R^1$ Fluor oder Trifluormethyl und

R Hydroxy bedeutet,

lassen sich nach dem unter 1 (oben) angegebenen Verfahren herstellen.

Verwendet man im Verfahren 1 beispielsweise 3,3-Dimethyl-4-(2,2-dichlor-3,3-trifluorpropyl)-2-chlor-1-cyclobutanon als Ausgangsstoff, so kann der Reaktionsverlauf durch das folgende Formelschema wiedergegeben werden:

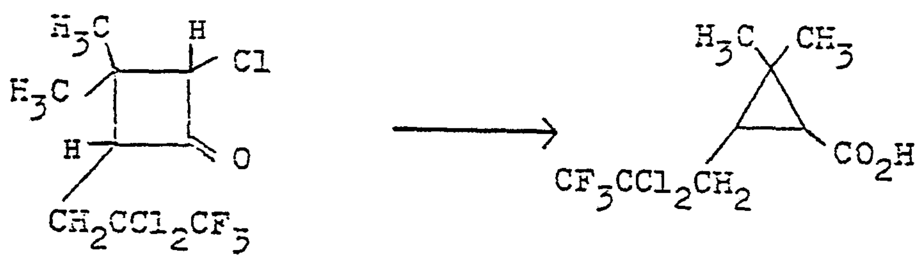

Die als Ausgangsprodukte zu verwendenden Cyclobutanone der Formel II sind neu. Im einzelnen seien genannt:

3,3-Dimethyl-4-(2,2-dichlor-3,3,3-trifluorpropyl)-2-chlor-1-cyclobutanon,
3,3-Dimethyl-4-(2,2-dichlor-3,3,4,4,4-pentafluorbutyl)-2-chlor-1-cyclobutanon.

Die bei Verfahren 1 durchzuführende, im Prinzip bekannte Favorskii-Umlagerung erfolgt in Gegenwart wäßriger Basen, die äquimolar eingesetzt werden. Als Beispiele seien genannt:
Natrium-, Kalium-, Calciumhydroxid, Kalium- und Natriumcarbonat. Besonders bevorzugt ist Natriumhydroxid.

Die Temperatur liegt zwischen -20 und +40°C, bevorzugt ist +10 bis +25°C.. Ferner wird in Gegenwart eines inerten Verdünnungsmittels gearbeitet. Hier kommen beispielsweise Ether wie Dioxan, Tetrahydrofuran, Dimethoxyethan in Frage. Es können jedoch auch nicht mit Wasser mischbare Lösungsmittel, wie Methylenchlorid, Petrolether, Benzol, Toluol, Chlorbenzol verwendet werden. Nach beendeter Reaktion wird die Säure durch Ansäuern mit Mineralsäuren wie Salzsäure oder Schwefelsäure in Freiheit gesetzt.

Nach dem erfindungsgemäßen Verfahren fallen ganz überwiegend die cis-Isomeren Säuren an, die wirksamere Ester liefern. Es sind zwar mehrere sehr ähnliche Reaktionen wie das erfindungsgemäße Verfahren bekannt (DE-OS

2 539 048, 2 654 062, 2 813 337); es zeigte sich jedoch, daß im Falle der erfindungsgemäßen neuen Ausgangsstoffe der Formel II spezielle Reaktionsbedingungen gefunden werden mußten, um Nebenreaktionen zu vermeiden.

Eine der wichtigsten Nebenreaktionen der Favorskii-Umlagerung ist die nucleophile Substitution (vgl. J.M.Conia u. J.R.Salem, Accounts of Chemical Research Vol. 5, 1972, Seite 33);

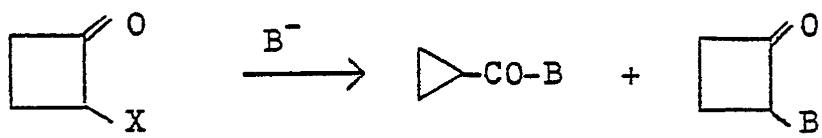


X = Br, Cl, OTs

Es ist literaturbekannt (Acc. of Chem. Res. 1972, Seite 34), daß im Gegensatz zu den α-Brom-cyclobutanonen die α-Chlorcyclobutanone nicht spezifisch reagieren und hauptsächlich zum Substitutionsprodukt führen.

So beträgt beispielsweise auch die Ausbeute der Reaktion

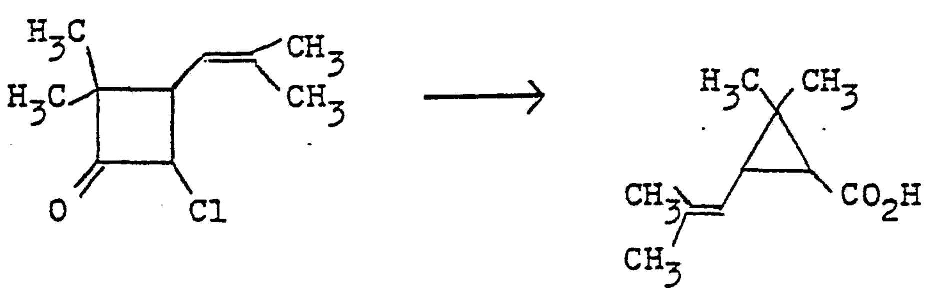

nur 25% (DT-OS 2 539 o48).

Daß auch die Fluorsubstitution eine entscheidende Rolle spielt, ersieht man daraus, daß beim Erhitzen mit wässrigen Basen der fluorhaltige Rest abgespalten wird.

Wesentlich ist auch, daß man im Gegensatz zu den üblichen Verfahrensweisen nicht in Gegenwart von Alkoholen oder Alkoholat arbeiten darf, da sonst Nebenreaktionen auftreten.
So erhält man beispielsweise in Gegenwart von Alkoholen Gemische aus dem Substitutionsprodukt und dem gewünschten Cyclopropanderivat. Beim Arbeiten mit Methylat entstehen komplexe Substanzgemische, die nur zum Teil aufgeklärt werden konnten. So entstehen z.B. in der Seitenkette substituierte Produkte, wie z.B.

$$R^1CF_2-\underset{OCH_3}{\overset{Cl}{C}}-CH_2- \quad .$$

Durch die bei uns gewählten Reaktionsbedingungen kann verhindert werden, daß die Cyclopropancarbonsäuren der Formel I durch Nebenprodukte verunreinigt werden. Es wird außerdem erreicht, daß sie überwiegend in der cis-Form anfallen. Beide Ergebnisse waren überraschend.

Die neuen Cyclobutanone der Formel II werden nach Verfahren 3 (oben) erhalten.

Verwendet man beispielsweise bei Verfahren 3 3,3-Dimethyl-4-chlor-4-(2,2-dichlor-3,3,3-trifluorpropyl)-1-cyclobutanon als Ausgangsstoff, so kann der Reaktionsablauf durch das folgende Formelschema wiedergegeben werden:

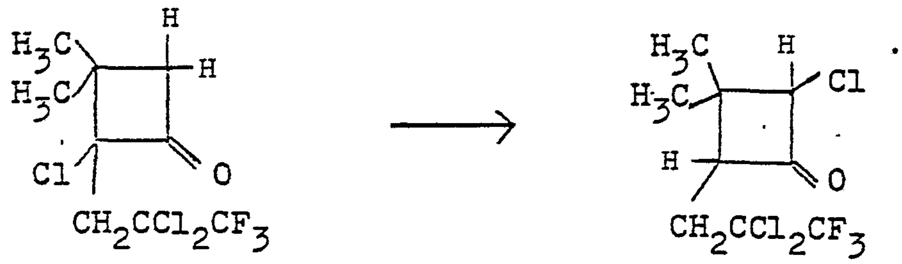

Die als Ausgangsprodukte zu verwendenden Cyclobutanone der Formel III sind neu. Im einzelnen seien genannt:
3,3-Dimethyl-4-chlor-4-(2,2-dichlor-3,3,3-trifluorpropyl)-1-cyclobutanon,
3,3-Dimethyl-4-chlor-(2,2-dichlor-3,3,4,4,4-pentafluorbutyl)-1-cyclobutanon.

Die Reaktion ist im Prinzip literaturbekannt (J.A.C.S. 101, 5853, 1979).

Dabei wird die Umlagerung mit Triethylamin durchgeführt. Dies ergibt bei 120°C in 15 Stunden nur ca. 30 % umlagertes Produkt.

Es wurde gefunden, daß im Falle der vorliegenden Cyclobutanone der Formel III die Umlagerung bereits nach kurzer Zeit quantitativ ist, wenn mit quartären Ammoniumsalzen als Katalysator gearbeitet wird. Gegebenenfalls kann auch ein Verdünnungsmittel zugesetzt werden, dies ist jedoch nicht notwendig. Bevorzugt wird in der Schmelze gearbeitet. Der Temperaturbereich liegt zwischen 50 und 200°C, vorzugsweise zwischen 100 und 150°C. Eine weitere Aufarbeitung ist nicht notwendig. Das verwendete quartäre Ammoniumsalz wirkt sich nicht störend auf die Folgereaktionen aus. Die Cyclobutanone der Formel II können jedoch auch durch Umkristallisation oder durch Destillieren gereinigt werden.

Als quartäre Ammoniumsalze können alle üblichen Phasentransferkatalysatoren verwendet werden. Besonders bevorzugt sind Tetrabutylammoniumchlorid oder -bromid, Triethylbenzylammoniumchlorid und Methyltrioctylammoniumchlorid.

Die Cyclobutanone der Formel III werden nach Verfahren 5 (oben) erhalten.

Verwendet man beispielsweise 5,5,6,6,6-Pentafluor-2,4,4-trichlor-hexansäurechlorid als Ausgangsstoff, so kann der Reaktionsverlauf durch das folgende Formelschema wiedergegeben werden:

$CF_3-CF_2-CCl_2-CH_2-CHCl-CO-Cl$

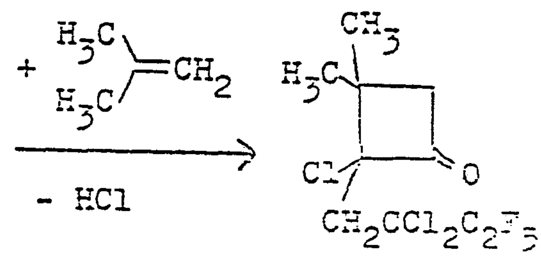

Die als Ausgangsprodukte zu verwendenden Säuren bzw. Säurehalogenide sind neu. Als Beispiele seien genannt: 5,5,5-Trifluor-2,4,4-trichlor-pentansäurechlorid, 5,5,6,6,6-Petafluor-2,4,4-trichlor-hexansäurechlorid.

Die Säurehalogenide werden aus den entsprechenden Säuren durch allgemein bekannte und übliche Methoden, beispielsweise durch Reaktion mit Phosgen oder Thionylchlorid erhalten.

Verfahren 5 läuft in der Weise ab, daß aus dem Säurehalogenid der Formel IV Halogenwasserstoff in einem Verdünnungsmittel mit Hilfe von Basen abgespalten wird und das entstandene Keten mit Isobutylen zur Umsetzung gebracht wird.

Als Basen sind Amine geeignet, vorzugsweise Triethylamin.

Als Verdünnungsmittel sind vorzugsweise Petrolether, Benzin, Cyclopentan, Cyclohexan, Toluol, Xylol oder Chlorbenzol verwendbar.

Die Reaktionstemperatur liegt zwischen 0 und 15o°C, vorzugsweise zwischen 15 und 1oo°C.

Die den neuen Säurehalogeniden der Formel IV zugrundeliegenden neuen Säuren der Formel VI

$$R^1-CF_2CCl_2CH_2CHCl-CO-X \qquad (VI)$$

in welcher

$R^1$ für Fluor oder Trifluormethyl steht und

X OH bedeutet,

werden nach Verfahren 7 (oben) erhalten.

Verwendet man beispielsweise 1,1,1-Trifluor-2,2,2-trichlorethan als Ausgangsstoff, so kann der Reaktionsverlauf durch das folgende Formelschema wiedergegeben werden:

$$CF_3CCl_3 + CH_2=CH-CO_2H \longrightarrow CF_3CCl_2CH_2CHCl-CO_2H$$

Die als Ausgangsprodukte zu verwendenden Produkte der Formel V sind bekannt. Es seien genannt: 1,1,1-Trifluor-2,2,2-trichlorethan und 1,1,1,2,2-Pentanfluor-3,3,3-trichlorpropan.

Die Addition an Acrylsäure erfolgt mit Hilfe von Katalysatoren. Am besten bewährt hat sich Kupfer(I)chlorid. Als Verdünnungsmittel wird bevorzugt ein polares Lösungsmittel wie Dimethylformamid, Dimethylacetamid oder Acetonitril verwendet. Besonders bevorzugt ist Acetonitril.

Die Temperatur liegt bei 5o-2oo°C, bevorzugt zwischen 1oo und 15o°C, wobei mindestens ein Druck von 5 bar erreicht werden sollte. Vorzugsweise wird bei einem Anfangsdruck zwischen 5 und 2o bar gearbeitet, der durch Aufpressen von Stickstoff eingestellt wird.

Die Cyclopropancarbonsäuren und ihre Derivate der Formel I lassen sich in an sich bekannter Weise in ihre Ester z.B. den Pentafluorbenzyl- oder den 3-Phenoxy-4-fluorbenzylester überführen. Anschließende Dehydrohalogenierung z.B. in Anwesenheit von Alkalicarbonaten in aprotischen polaren Verdünnungsmitteln sowie in Anwesenheit von quartären Ammoniumsalzen oder in Dimethylformamid in Anwesenheit von Kaliumfluorid führt zu den Estern der Säuren:

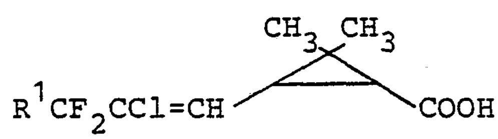

wobei

$R^1$ für Fluor oder $CF_3$ steht.

Diese Ester sind bekannte gute Schädlingsbekämpfungsmittel (DE-A 2 831 193, DE-A 2 907 609).

Beispiel 1

Herstellung von 5,5,5-Trifluor-2,4,4-trichlor-pentansäure.

In einem Autoklaven wird eine Mischung aus 18o g (2,5 Mol) Acrylsäure, 937,5 g (5 Mol) Trifluortrichlorethan, 75o ml Acetonitril und 15 g Kupfer(I)chlorid 24 Stunden auf 12o°C erhitzt. Der Anfangsdruck wurde durch Aufdrücken von Stickstoff auf 5 bar eingestellt. Nach dem Abkühlen wird abfiltriert und das Filtrat fraktioniert destilliert. Die Fraktion vom Siedepunkt 75-85°C/o,5 mbar besteht hauptsächlich aus der gewünschten 5,5,5-Trifluor-2,4,4-trichlor-pentansäure und wird direkt ins Säurechlorid umgewandelt:

Beispiel 2

Herstellung von 5,5,5-Trifluor-2,4,4-trichlor-pentansäurechlorid.

223 g rohe 5,5,5-Trifluor-2,4,4-trichlorpentansäure (aus Beispiel 1) werden zu 5oo ml Thionylchlorid getropft. Es wird solange am Rückfluß erhitzt, bis die Gasentwicklung aufhört. Man erhält 146 g 5,5,5-Trifluor-2,4,4-trichlorpentansäurechlorid vom Kp = 61-62°C/18 mbar.

Beispiel 3

Herstellung von 2-Chlor-2-(2,2-dichlor-3,3,3-trifluorpropyl-3,3-dimethyl-cyclobutanon

83,4 g (o,3 Mol) 5,5,5-Trifluor-2,4,4-trichlorpentansäurechlorid werden in einem 1,3 l Autoklaven in 36o ml Cyclohexan vorgelegt. Dann werden 2oo g Isobutylen aufgepreßt, der Autoklav verschlossen und auf 65°C geheizt. Dann werden innerhalb von 2 Stunden 3o,6 g Triethylamin in 3oo ml Cyclohexan zugepumpt. Nach beendetem Zupumpen hält man noch 4 Stunden auf 65°C und läßt dann abkühlen. Nach Abfiltrieren des Triethylaminhydrochlorids wird das Cyclohexan abdestilliert. Man erhält 54,5 g 2-Chlor-2-(2,2-dichlor-3,3,3-trifluor)-propyl-3,3-dimethyl-cyclobutanon vom Schmelzpunkt 49-51°C (Ausbeute 61% der Theorie).

$^1$H-NMR ($CDCl_3$): 1,4 und 1,5 ppm (2 $CH_3$), 2,7-3,45 ppm (AB-System, $CH_2$), 3,o5 ppm (S, $CH_2$).

<u>Beispiel 4</u>

Herstellung von 2-(2,2-Dichlor-3,3,3-trifluor)-propyl-3,3-dimethyl-4-chlor-cyclobutanon

Man vermischt 5o g (o,168 Mol) 2-Chlor-2-(2,2-dichlor-3,3,3-trifluor)-propyl-3,3-dimethyl-cyclobutanon mit 4,7 g Tetrabutylammoniumchlorid und erhitzt 6 Stunden auf 125°C. Nach dem Abkühlen wird in Methylenchlorid aufgenommen, dreimal mit Wasser gewaschen, mit Natriumsulfat getrocknet, abfiltriert und eingeengt. Man erhält 46,6 g (93,5 % der Theorie) 2-(2,2-Dichlor-3,3,3-trifluor)-propyl-3,3-dimethyl-4-chlor-cyclobutanon vom Schmelzpunkt 46-48°C.

$^1$H-NMR ($CDCl_3$): 1,o5 und 1,6 ppm (2 $CH_3$), 2,1-3,o ppm (m, $CH_2$), 3,5 ppm (m, CH), 4,8 ppm (d, CH).

Beispiel 5

Herstellung von cis/trans-2,2-Dimethyl-3-(2,2-dichlor-3,3,3-trifluor)propyl-cyclopropancarbonsäure

8,92 g (o,o3 Mol) 2-(2,2-Dichlor-3,3,3-trifluor)-propyl-3,3-dimethyl-4-chlor-cyclobutanon werden in 45 ml 1o%iger Natronlauge suspendiert. Nach Zugabe von 5 ml Dioxan rührt man 12 Stunden bei Raumtemperatur. Man säuert mit Salzsäure an und extrahiert dreimal mit Methylenchlorid. Nach Trocknen mit Natriumsulfat und Abdestillieren des Lösungsmittels erhält man 7,9 g (94,5 % der Theorie) cis/trans-2,2-Dimethyl-3-(2,2-dichlor-3,3,3-trifluor)-propyl-cyclopropancarbonsäure vom Schmelzpunkt 66-68°C. Cis/trans-Verhältnis: 72%cis, 28% trans.

Beispiel 6

Herstellung von cis/trans-2,2-Dimethyl-3-(2,2-dichlor-3,3,3-trifluor)-propyl-cyclopropancarbonsäurechlorid

27,9 g (o,1 Mol) cis/trans-2,2-Dimethyl-3-(2,2-dichlor-3,3,3-trifluorpropyl)-cyclopropancarbonsäure werden in 2oo ml Tetrachlorkohlenstoff gelöst und mit 3o g Thionylchlorid versetzt. Nach einstündigem Rückflußkochen läßt man abkühlen und engt am Rotationsverdampfer ein. Das zurückbleibende Säurechlorid wird durch Destillation gereinigt: Kp = 68-72°C/o,2 mbar.

Beispiel 7

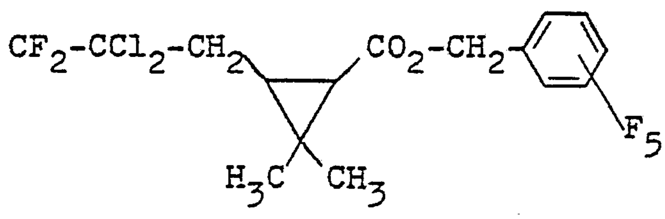

3 g cis/trans-2,2-Dimethyl-3-(2,2-dichlor-3,3,3-trifluorpropyl)-cyclopropancarbonsäurechlorid werden mit 1oo ml Toluol und 2 g Pentafluorbenzylalkohol vermischt. Dann tropft man bei Raumtemperatur unter Rühren o,81 g Pyridin in 5o ml Toluol zu und rührt 4 Stunden bei Raumtemperatur nach. Dann wird das Reaktionsgemisch mit 15oml Wasser versetzt, die organische Phase abgetrennt und mit 1oo ml Wasser gewaschen. Die organische Phase wird mit Natriumsulfat getrocknet, abfiltriert, einrotiert und bei 6o°C im Hochvakuum andestilliert. Der Rückstand besteht aus cis/trans-2,2-Dimethyl-3-(2,2-dichlor-3,3,3-trifluorpropyl)-cyclopropancarbonsäure-pentafluorbenzylester.

Beispiel 8

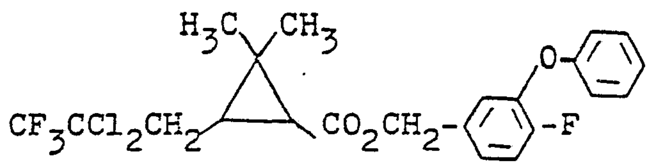

Analog Beispiel 7 werden aus 3 g cis/trans-2,2-Dimethyl-3-(2,2-dichlor-3,3,3-trifluorpropyl)-cyclopropancarbonsäurechlorid, 2,2 g 4-Fluor-3-phenoxybenzylalkohol und o,8 g Pyridin cis/trans-2,2-Dimethyl-3-(2,2-dichlor-3,3,3-trifluorpropyl)-cyclopropancarbonsäure-4'-fluor-3'-phenoxy-benzylester erhalten.

Beispiel 9

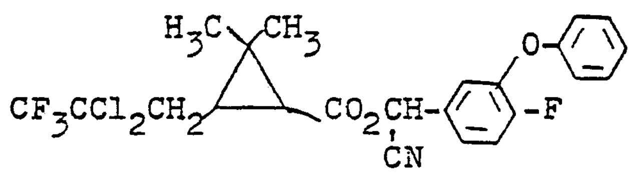

Analog Beispiel 7 werden aus 3 g cis/trans-2,2-Dimethyl-3-(2,2-dichlor-3,3,3-trifluorpropyl)-cyclopropancarbonsäurechlorid, 2,4 g 4-Fluor-3-phenoxy-α-cyano-benzylalkohol und o,8 g Pyridin cis/trans-2,2-Dimethyl-3-(2,2-dichlor-3,3,3-trifluorpropyl)-cyclopropancarbonsäure-4'-fluor-3'-phenoxy-α'-cyano-benzylester erhalten.

Beispiel 1o

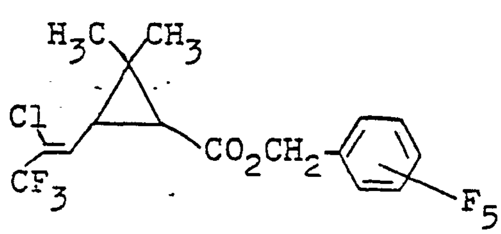

2,32 g cis/trans-2,2-Dimethyl-3-(2,2-dichlor-3,3,3,-trifluorpropyl)-cyclopropancarbonsäure-pentafluor-

benzylester werden in 1o ml Dimethylformamid gelöst und mit o,8 g 1,8-Diazabicyclo(5.4.o)undec-7-en versetzt und 3 Stunden auf 11o°C erhitzt. Nach dem Abkühlen wird mit Wasser verdünnt und mit konz. Salzsäure schwach sauer gestellt, dann sofort dreimal mit Ether extrahiert und die Etherlösung mit konz. Kochsalzlösung neutral gewaschen. Die organische Phase wird mit Natriumsulfat getrocknet, abfiltriert, einrotiert und im Hochvakuum ca. 1 Stunde andestilliert. Der Wirkstoff kann auch durch Destillation gereinigt werden: Kp = 136-145°C/ o,1 mbar.

Beispiel 11

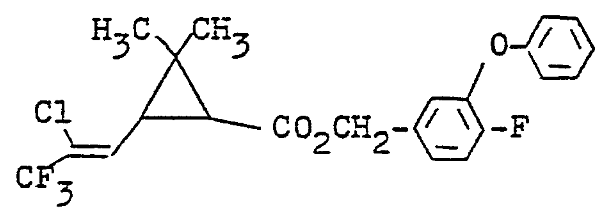

4 g cis/trans-2,2-Dimethyl-3-(2,2-dichlor-3,3,3-trifluorpropyl)-cyclopropancarbonsäure-4'-fluor-3'-phenoxy-benzylester werden in 2o ml Acetonitril gelöst. Man gibt 4 g Kaliumcarbonat und o,5 g Tetrabutylammoniumbromid zu und erhitzt im Autoklaven 4 Stunden auf 18o°C. Nach dem Abkühlen wird mit Wasser verdünnt und mit Salzsäure neutral gestellt. Man extrahiert mit Methylenchlorid, trocknet mit Natriumsulfat, engt am Rotationsverdampfer ein und destilliert bei 6o°C im Hochvakuum an. Man erhält 3,4 g cis/trans-2,2-Dimethyl-3-(2-chlor-3,3,3-trifluorpropenyl)-cyclopropancarbonsäure-4'-fluor-3'-phenoxy-benzylester, wobei die Z-Isomeren ganz stark überwiegen. Brechungsindex: $n_D^{20}$=1,519.

Beispiel 12

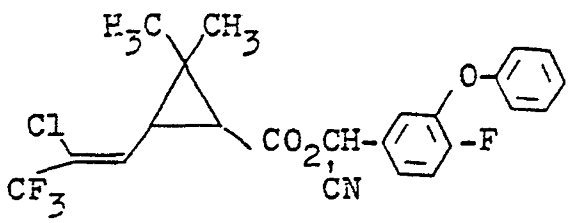

Analog Beispiel 1o wird aus 3,9 g cis/trans-2,2-Dimethyl-3-(2,2-dichlor-3,3,3-trifluorpropyl)-cyclopropancarbonsäure-4'-fluor-3'-phenoxy- α'-cyano-benzylester 3,5 g cis/trans-2,2-Dimethyl-3-(2-chlor-3,3,3-trifluorpropenyl)-cyclopropancarbonsäure-4'-fluor-3'-phenoxy- α'-cyano-benzylester erhalten.

Beispiel 13

Herstellung von 5,5,6,6,6-Pentafluor-2,4,4-trichlor-hexansäurechlorid.

In einem Autoklaven wird eine Mischung aus 18o g (2,5 Mol) Acrylsäure,1185 g (5 Mol) 1,1,1,2,2-Pentafluor-3,3,3-trichlorpropan, 75o ml Acetonitril und 15 g Kupfer-(I)chlorid 48 Stunden auf 12o°C erhitzt. Der Anfangsdruck wurde auf 7 bar eingestellt. Nach dem Abkühlen wird abfiltriert und das Filtrat fraktioniert destilliert. Die Fraktion vom Kp = 1o2-11o°C/o,2 mbar besteht zum größten Teil aus 5,5,6,6,6-Trifluor-2,4,4-trichlorhexansäure. Die Fraktion wird zu 5oo ml Thionylchlorid getropft und 1 Stunde am Rückfluß erhitzt. Das 5,5,6,6,6-Pentafluor-2,4,4-trichlor-hexansäurechlorid siedet bei 88-9o°C/ 15 mbar.

Beispiel 14

Herstellung von 2-Chlor-2-(2,2-dichloro-3,3,4,4,4-pentafluor-butyl)-3,3-dimethyl-cyclobutanon.

98,4 g (o,3 Mol) 5,5,6,6,6-Pentafluor-2,4,4-trichlor-pentansäurechlorid werden in einem 1,3 l-Autoklaven in 36o ml Cyclohexan vorgelegt. Dann werden 2oo g Isobuten aufgepreßt und auf 65°C geheizt. Dann werden innerhalb von 2 Stunden 3o,6 g Triethylamin in 3oo ml Cyclohexan zugepumpt. Nach beendetem Zupumpen hält man noch 5 Stunden auf 6o-65°C und läßt dann abkühlen. Nach Abfiltration des Triethylaminhydrochlorids wird das Cyclohexan abdestilliert. Man erhält einen öligen Rückstand, der durch Destillation im Hochvakuum gereinigt wird. (Kugelrohr; Kp = 1oo°C/o,3 mbar).

Beispiel 15

Herstellung von 3,3-Dimethyl-2-(2,2-dichlor-3,3,4,4,4-pentafluor)-propyl-4-chlor-1-cyclobutanon.

Man vermischt 2o g 3,3-Dimethyl-2-chlor-2-(2,2-dichlor-3,3,4,4,4-pentafluor)-propyl-1-cyclobutanon mit 2 g Tetrabutylammoniumbromid und erhitzt 6 Stunden auf 125°C. Nach dem Abkühlen wird der Kolbeninhalt direkt in die nächste Stufe eingesetzt (Beispiel 16).

Beispiel 16

Analog Beispiel 5 erhält man rohe 2,2-Dimethyl-3-(2,2-dichlor-3,3,4,4,4-pentafluor)-butyl-cyclopropancarbonsäure als Öl, die auf der Stufe des Säurechlorids gereinigt wird (Beispiel 17).

Beispiel 17

Herstellung von cis/trans-2,2-Dimethyl-3-(2,2-dichlor-3,3,4,4,4-pentafluor)-butyl-cyclopropancarbonsäure-chlorid.

2o g rohe 2,2-Dimethyl-3-(2,2-dichlor-3,3,4,4,4-pentafluor)-butyl-cyclopropancarbonsäure werden in 2oo ml Tetrachlorkohlenstoff gelöst und mit 25 ml Thionylchlorid versetzt. Nach einstündigem Rückflußkochen läßt man abkühlen und engt am Rotationsverdampfer ein. Das zurückbleibende Säurechlorid wird durch Destillation gereinigt. Kp. = 78-84°C/o,3 mbar. cis/trans-Verhältnis 74:26.

Beispiel 18

Analog Beispiel 7 werden folgende Verbindungen erhalten:

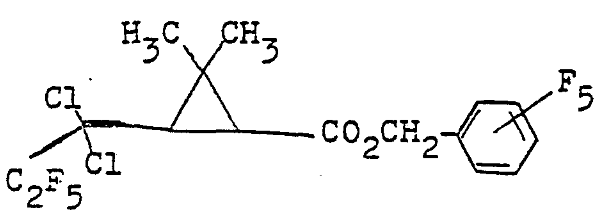

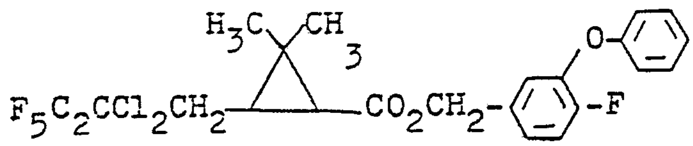

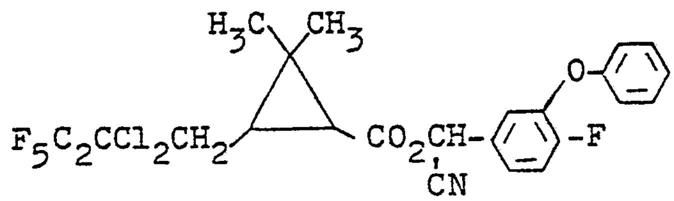

Beispiel 19

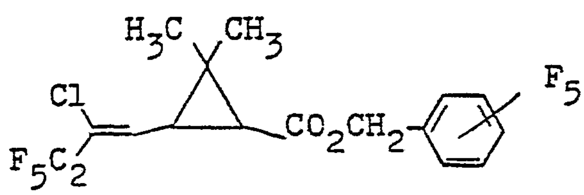

1,o2 g (2 mMol) cis/trans-2,2-Dimethyl-3-(2,2-dichlor-3,3,4,4,4-pentafluor)-butyl-cyclopropancarbonsäure-pentafluorbenzylester werden in 7 ml Dimethylacetamid gelöst und mit o,3 g 1,8-Diazabicyclo(5.4.o)undec-7-en versetzt und 4 Stunden auf 1oo°C erhitzt. Nach dem Abkühlen wird mit Wasser verdünnt und mit konz. Salzsäure schwach sauer gestellt, dann sofort dreimal mit Ether extrahiert und die vereinigten Etherauszüge mit konz. Kochsalzlösung neutral gewaschen. Die organische Phase wird mit Natriumsulfat getrocknet, abfiltriert, einrotiert und im Hochvakuum andestilliert. Der Rückstand besteht aus cis/trans-2,2-Dimethyl-3-(2-chlor-3,3,4,4,4-pentafluoro-but-1-enyl)-cyclopropancarbonsäurepentafluorbenzylester.

Beispiel 2o

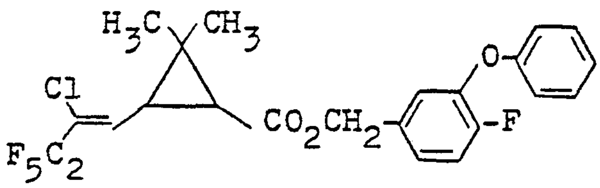

2 g cis/trans-2,2-Dimethyl-3-(2,2-dichlor-3,3,4,4,4-pentafluor)-butyl-cyclopropancarbonsäure-4'-fluor-3'-phenoxy-benzylester werden in 1o ml Acetonitril gelöst. Man gibt 1,5 g Kaliumcarbonat (feingepulvert) und o,3 g Tetrabutylammoniumchlorid zu und erhitzt im Autoklaven 12 Stunden auf 17o°C. Die Aufarbeitung erfolgt analog Beispiel 11. Man erhält 1,6g cis/trans-2,2-Dimethyl-3-(2-chlor-3,3,4,4,4-pentafluor-butenyl)-cyclopropancarbonsäure-4'-fluor-3'-phenoxy-benzylester.

Beispiel 21

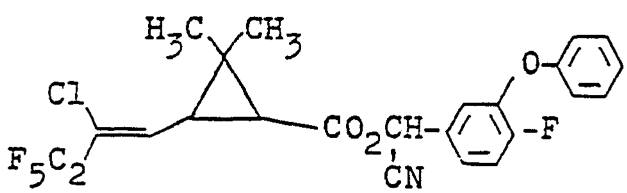

2,9 g cis/trans-2,2-Dimethyl-3-(2,2-dichlor-3,3,4,4,4-pentafluor)-butyl-cyclopropancarbonsäure-4'-fluor-3'-phenoxy-α'-cyano-benzylester werden in 1o ml Dimethylformamid gelöst. Man gibt o,7 g gut getrocknetes Kaliumfluorid hinzu und erhitzt eine Stunde auf 15o°C. Nach dem Abkühlen wird mit Wasser verdünnt und neutral gestellt. Nach dreimaligem Extrahieren mit Ether werden

die vereinigten Etherauszüge getrocknet und eingeengt. Der entstandene 2,2-Dimethyl-3-(2-chlor-3,3,4,4,4-pentafluor)-butenyl-cyclopropancarbonsäure-4'-fluor-3'-phenoxy-α'-cyano-benzylester wird chromatographisch (Kieselgel/n-Hexan/Chloroform 3:1) gereinigt.

Patentansprüche

1. Verfahren zur Herstellung von chlor-fluor-alkylsubstituierten Cyclopropancarbonsäuren und deren Derivaten der Formel I

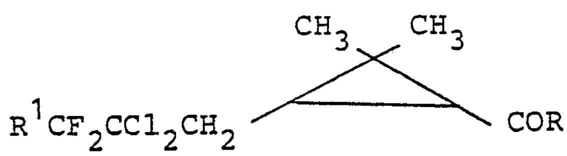

(I)

in welcher

$R^1$ für Fluor oder $CF_3$ steht und

R für OH, Halogen, $C_{1-4}$-Alkoxy steht,

dadurch gekennzeichnet, daß man chlor-fluor-alkylsubstituierte Halogencyclobutanone der Formel II

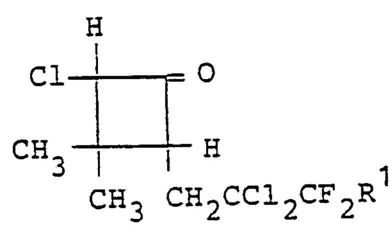

(II)

in welcher

$R^1$ die oben angegebene Bedeutung hat,

in wasserhaltigen aprotischen organischen Verdünnungsmitteln bei -20 bis +40°C mit wäßrigen Basen umsetzt und aus den dabei erhaltenen Salzen die freie Säure bzw. deren Derivate in üblicher Weise herstellt.

2. Chlor-fluor-alkylsubstituierte Cyclopropancarbonsäuren und ihre Derivate der Formel I

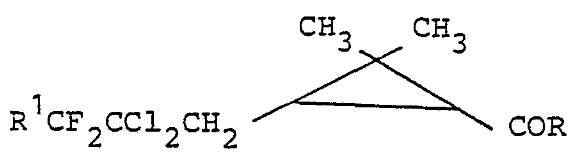

(I)

in welcher

$R^1$ für $CF_3$ steht, und

R die in Anspruch 1 angegebene Bedeutung hat.

3. Chlor-fluoralkylsubstituierte Halogencyclobutanone der Formel II

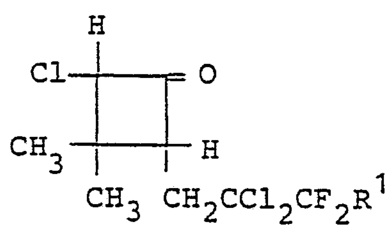

(II)

in welcher

$R^1$ für Fluor oder $CF_3$ steht.

4. Verfahren zur Herstellung der Cyclobutanone der Formel II

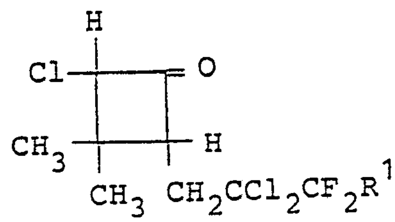

(II)

in welcher

$R^1$ die oben angegebene Bedeutung hat,

dadurch gekennzeichnet, daß man die Cyclobutanone der Formel III

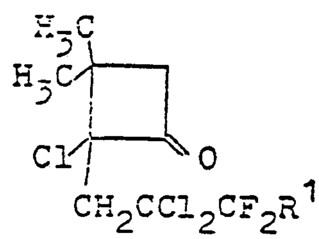

(III)

in welcher

$R^1$ die oben angegebene Bedeutung besitzt,

in Gegenwart eines quartären Ammoniumsalzes erhitzt.

5. Cyclobutanone der Formel III

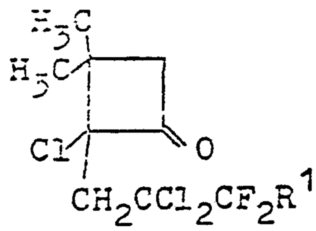

(III)

in welcher

$R^1$ die oben angegebene Bedeutung besitzt.

6. Verfahren zur Herstellung der Cyclobutanone der Formel III

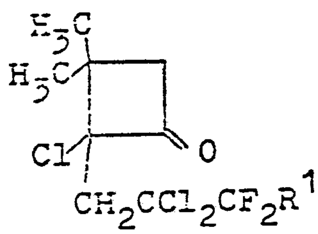

(III)

in welcher

$R^1$ die oben angegebene Bedeutung besitzt,

dadurch gekennzeichnet, daß man Isobutylen mit einem Säurehalogenid der Formel IV

$$R^1-CF_2-CCl_2-CH_2-CHCl-CO-X \qquad (IV)$$

in welcher

$R^1$ für Fluor oder Trifluormethyl steht und

X Chlor oder Brom bedeutet,

in Gegenwart eines Verdünnungsmittels und in Gegenwart eines tertiären Amins zur Umsetzung bringt.

7. Säurehalogenide der Formel IV

$$R^1-CF_2-CCl_2-CH_2-CHCl-CO-X \quad (IV)$$

in welcher

$R^1$ für Fluor oder Trifluormethyl steht und

X Chlor oder Brom bedeutet.

8. Verfahren zur Herstellung der Säurehalogenide der Formel IV

$$R^1-CF_2-CCl_2-CH_2-CHCl-CO-X \quad (IV)$$

in welcher

$R^1$ für Fluor oder Trifluormethyl steht und

X Chlor oder Brom bedeutet,

dadurch gekennzeichnet, daß man Verbindungen der Formel V

$$R^1\text{-}CF_2\text{-}CCl_3 \qquad (V)$$

in welcher

$R^1$ die oben angegebene Bedeutung hat,

in Gegenwart von Katalysatoren an Acrylsäure addiert und die entstandene Säure in üblicher Weise in das Halogenid (X = Cl, Br) überführt.